# EUROPÄISCHE PATENTANMELDUNG

(11) **EP 4 166 119 A1**
(43) Veröffentlichungstag der Anmeldung: **19.04.2023**
(21) Anmeldenummer: 21203087.8
(22) Anmeldetag: 18.10.2021
(51) Int. Cl.: A61F 13/06, A61F 13/08, A61F 13/10, A61F 13/14

(54) **POSTOPERATIVER KÖRPERVERBAND**

(71) Anmelder: Sahin, Gülkan, 9000 St. Gallen (CH)
(72) Erfinder: Sahin, Gülkan, 9000 St. Gallen (CH)
(74) Vertreter: IPrime Rentsch Kaelin AG

(57) **Zusammenfassung**

Ein Körperverband (1, 10), insbesondere für die postoperative Behandlung nach einer Liposuktion, umfassend mindestens eine erste flüssigkeitsdurchlässige und/oder flüssigkeitsabsorbierende Innenschicht (100), mindestens eine zweite flüssigkeitsabsorbierende Zwischenschicht (200) und mindestens eine dritte flüssigkeitsundurchlässige Aussenschicht (300), dadurch gekennzeichnet, dass der Körperverband einteilig ist und dazu geeignet ist, einen Körperbereich eines Patienten zu umschliessen. Der Körperverband weist mindestens eine Öffnung auf, durch welche bei der vorgesehenen Benutzung des Körperverbandes ein Körperteil des Patienten ragen kann und wobei an der mindestens einen Öffnung des Körperverbandes Abdichtungselemente (3, 4, 30, 40) vorgesehen sind, die dazu geeignet sind, den Körperverband gegenüber dem aus der Öffnung ragenden Körperteil des Patienten dichtend abzuschliessen.

## Beschreibung

### Gebiet der Technik

Die vorliegende Erfindung bezieht sich auf einen Körperverband, insbesondere zur postoperativen Behandlung nach Liposuktionen, gemäss dem Oberbegriff des unabhängigen Patentanspruchs.

### Technologischer Hintergrund

Aus dem Stand der Technik sind verschiedene Vorrichtungen und Lösungen für postoperative Pflege nach Liposuktionen und ähnlichen Eingriffen bekannt. Liposuktionen zählen in der plastischen Chirurgie heute zu den am häufigsten durchgeführten Eingriffen, wobei man zwischen sogenannter trockener Liposuktionstechnik ohne Lösungsinjektion und nasser Liposuktionstechnik mit Lösungsinjektion unterscheidet. Letztere ist häufig die bevorzugte Variante, da diese über mehrere pflegerelevante Vorteile verfügt, zum Beispiel können interne und externe Blutungen sowie der Bedarf für eine Vollnarkose reduziert werden. Während der nassen Liposuktion wird eine fettauflösende Flüssigkeit in die Unterhaut injiziert, eine sogenannte Tumeszenzlösung, welche anschliessend entweder von den Lymphgefässen des Patienten aufgenommen wird oder aus den Einschnittstellen der Liposuktion über mehrere Tage bis Wochen ausläuft. Die Tumeszenzlösung besteht aus einem physiologischen Mittel, Adrenalin und optional einem Betäubungsmittel. Hierbei führt das Adrenalin zu einer Kontraktion der Blutgefässe, wobei das physiologische Mittel, oft eine Kochsalzlösung, das Fettgewebe verarbeitet und homogenisiert, um ein einfacheres Absaugen des Fettgewebes zu erzielen. Das beabsichtigte Auslaufen der Flüssigkeit aus den Einschnittstellen wird generell bevorzugt, da so die Genesungsdauer verkürzt und die Entzündungsgefahr reduziert werden kann. Seltene, jedoch gelegentlich auftretende unerwünschte Folgen der trockenen Liposuktion sind Unregelmässigkeiten der Hautoberfläche, nämlich Dellenbildungen und/ oder Überlappen von Fettpolstern. Diese unerwünschten Nebenwirkungen können mittels der nassen Liposuktion weitgehend vermieden werden, jedoch ist eine postoperative Pflege für den Erfolg ausschlaggebend. Hier ist zu gewährleisten, dass das restliche Fettgewebe zusammen mit der injizierten Flüssigkeit aus dem Körper über die Einschnittstellen austritt.

Während der postoperativen Genesungsdauer wird es empfohlen, kontinuierlich einen Kompressionsverband zu tragen, da der Kompressionsverband konstant von aussen einen im Wesentlichen gleichmässigen Druck auf die behandelten Körperteile ausübt und das Auslaufen der injizierten Flüssigkeit aus den Einschnittstellen begünstigt. Da der Kompressionsverband über mehrere Tage bis Wochen kontinuierlich getragen werden sollte, ist dieser meistens aus einem atmungsaktiven und flüssigkeitsdurchlässigen Material. Um den Heilungsprozess zu fördern, sollte die Haut der Patienten trotz Kompressionsverband weiterhin gut atmen und Schweiss evaporieren können. Die genannten Materialerfordernisse des Kompressionsverbandes führen dazu, dass die postoperative Flüssigkeit oft unkontrolliert durch den Kompressionsverband austritt und in die Kleidung, Bettwäsche oder Weiteres des Patienten ausläuft und diese verschmutzen kann. Hinzu kommt regelmässig ein unangenehmes Körpergefühl auf der Haut aufgrund des Flüssigkeitsaustritts.

Bekannte Lösungen für die Handhabung der Leckage postoperativer Flüssigkeit aus Kompressionsverbänden und die daraus resultierende Verschmutzung der Kleidung, Bettwäsche oder Weiteres des Patienten, ist das Einbringen und Tragen absorptionsfähiger Verbände, Pflaster oder Einlagen unterhalb, innerhalb oder oberhalb des Kompressionsverbandes. Diese absorptionsfähigen Verbände, Pflaster und Einlagen haben jedoch einen negativen Einfluss auf die Atmungsfähigkeit sowie den Tragkomfort der Kompressionsverbände. Darüber hinaus ist ein Wechseln des Verbandes, Pflasters oder der Einlage nach einer gewissen Zeit notwendig, welches das empfohlene kontinuierliche Tragen des Kompressionsverbandes unterbricht und somit ein erhöhtes Entzündungsrisiko mit sich bringt.

Weitere Lösungen aus dem Stand der Technik sind formpassende mehrteilige Körperverbände, welche über den Kompressionsverband positioniert und mit Schnüren, Klettbandverschlüssen oder anderen Verschlüssen miteinander verbunden bzw. befestigt werden. Solche weisen ergänzend absorptionsfähige sowie flüssigkeitsundurchlässige Flächen auf. Die absorptionsfähige Fläche ist in diesen Beispielen dem Patienten zugewandt, während die flüssigkeitsundurchlässige Fläche dem Patienten abgewandt ist. Diese mehrteiligen Lösungen sind in der Lage einen Teil der austretenden Flüssigkeit zu absorbieren, können jedoch nicht die notwendige Dichtigkeit gewährleisten, die es benötigt, um das Durchnässen der Kleidung zu verhindern. Insbesondere an den Rändern des Körperverbandes kann Flüssigkeit austreten. Um diesen Effekt zu minimieren muss der Körperverband möglichst nahe an der Hautoberfläche des Patienten positioniert werden. Um den deshalb sehr eng anliegenden Körperverband überhaupt anlegen zu können, muss er wie oben beschrieben mehrteilig ausgestaltet sein. Ein solcher Körperverband ist unbequem zu tragen, und das Anlegen erfordert manuelles Geschick oder Hilfestellung durch Dritte.

Des Weiteren ist der Tragkomfort dieser Lösungen stark beeinträchtigt durch die enge Passform, ein Aufkleben auf der Hautoberfläche, die verwendeten Befestigungen und das Überlappen, Reiben und Scheuern von Schichten an den Befestigungs- und/oder Verbindungsstellen, bzw. Öffnungen, und gegebenenfalls an den Wunden selbst.

Das Auslaufen postoperativer Flüssigkeit nach einer Liposuktion ist häufig für Patienten nicht nur mit körperlichen Unannehmlichkeiten verbunden, sondern auch eine starke psychologische Belastung. Die Lösungen aus dem Stand der Technik bemühen sich nicht, die mit den postoperativen Flüssigkeitsauslauf verbundene psychologische Belastung zu mildern. Die oben genannten Verbände, Pflaster und Einlagen haben eine deutliche medizinische Ästhetik und fallen daher in der Öffentlichkeit unerwünscht auf. Sie vermögen den Patienten keine Sicherheit bezüglich eines Auslaufens zu vermitteln und bewirken den Eindruck eines «Krank-Seins».

Liposuktionen sind in den meisten Fällen mit einem Kliniksaufenthalt von einem Tag oder weniger verbunden, sodass ein Grossteil, der den Behandlungserfolg bestimmenden Genesung und die damit verbundene Kompressionstherapie anhand Kompressionsverbandes, im privaten Bereich bei den Patienten zuhause stattfindet. Das umständliche Wechseln und die Unannehmlichkeiten der Lösungen aus dem Stand der Technik tragen zu einer mangelhaften Selbstbehandlung bei. Es besteht daher ein Bedarf nach Lösungen, die die bereits erwähnten Nachteile des Standes der Technik vermeiden.

Das Dokument US 2011/0009845 A1 offenbart ein System und Verfahren für postoperative Pflege nach Liposuktionen. Die Erfindung umfasst eine Kompressionsbekleidung sowie eine oberhalb der Kompressionsbekleidung zu tragende mehrteilige postoperative Bekleidung. Die mehrteilige postoperative Bekleidung weist hier eine erste absorptionsfähige Schicht, eine zweite äussere flüssigkeitsresistente Schicht und Befestigungselemente auf. Die mehrteilige Bekleidung wird anhand der Befestigungselemente formpassend um den Körper im Bereich der behandelten Körperstellen befestigt. Hierbei ist vorgesehen, eine für die Flüssigkeitsaufnahme notwendige Dichtigkeit durch die enge Passform des Verbandes und der Befestigungselemente sicher zu stellen. Die enge Passform hat jedoch einen negativen Einfluss auf den Tragkomfort des Verbandes und auf die Beweglichkeit des Patienten während dem Tragen der postoperativen Bekleidung. Des Weiteren entstehen durch die Befestigungselemente entlang der Befestigungsstellen Überlappungen, sodass die Dichtigkeit an diesen Stellen nicht sichergestellt werden kann. Die Befestigungselemente können auch Reibungen bzw. Hautirritationen an den Befestigungsstellen verursachen, wobei diese wiederum den Heilungsprozess der Haut beeinträchtigen können. Ferner ist es sehr schwierig, bzw. beinahe unmöglich, eine sehr gut aufeinander abgestimmte bzw. dichte Verbindung der einzelnen Bestandteile der postoperativen Bekleidung anhand der Befestigungselemente zu realisieren. Zudem setzt dies erfindungsgemäss eine dem Patienten anliegende Tragweise voraus.

Das Dokument US 2010/0082007 A1 betrifft eine postoperative Bekleidung, umfassend einen Bekleidungskörper mit einer Innen- und einer Aussenseite, wobei der Bekleidungskörper an einem Körperbereich der Patienten positionierbar ist und einen im Wesentlichen gleichmässigen Druck auf den Körperbereich ausübt. Die postoperative Bekleidung umfasst ferner ein Befestigungselement auf einer Innenseite des Bekleidungskörpers, so ausgebildet, dass es mit einem weiteren Befestigungselement auf mindestens einer flüssigkeitsabsorbierenden Einlage zusammenwirkt und mit der mindestens einen Einlage lösbar verbunden ist. In dieser zitierten Erfindung wird die auslaufende Flüssigkeit von der mindestens einen absorptionsfähigen Einlage teilweise aufgenommen, jedoch scheitert die zitierte Erfindung vor allem bei grösseren Eingriffen, bzw. Liposuktionen, die auslaufende Flüssigkeit aufzunehmen. Ausserdem ist ein Wechseln der durchnässten Einlagen notwendig, wobei dieses Wechseln den im Wesentlichen gleichmässigen Druck auf den behandelten Körperteilen verändert. Dadurch unterbricht das notwendige Wechseln der Einlagen die für eine volle Genesung ausschlaggebende Kompressionstherapie. Auch die Dichtigkeit der postoperativen Bekleidung kann nicht kontinuierlich gewährleistet werden, da ein Wechseln der Einlagen auch einen Einfluss auf die Dichtigkeit der postoperativen Bekleidung hat. Diese Bekleidung hat zudem den bereits in Bezug auf den Stand der Technik erwähnten Nachteil der medizinischen Ästhetik und fällt daher in der Öffentlichkeit unerwünscht auf.

### Darstellung der Erfindung

Die folgende Beschreibung der vorteilhaften Ausführungsformen der Erfindung soll die Erfindung nicht auf einzelne vorteilhafte Ausführungsformen beschränken, sondern dient dazu dem Fachmann die Möglichkeit geben, die Erfindung herzustellen und zu verwenden.

Eine Aufgabe der vorliegenden Erfindung besteht darin mindestens einige der oben erwähnten Nachteile von postoperativen Pflegelösungen nach Liposuktionen und ähnlichen Eingriffen aus dem Stand der Technik zu vermeiden. Insbesondere besteht die Aufgabe der Erfindung darin, Patienten nach Liposuktionen und ähnlichen chirurgischen Eingriffen eine komfortable, ästhetisch ansprechende und effektive Lösung bereitzustellen, den postoperativen Flüssigkeitsausfluss aus Einschnittstellen kontrollierbar zu machen.

Diese und andere Aufgaben werden gelöst durch einen erfindungsgemässen Körperverband gemäss dem unabhängigen Anspruch. Weitere vorteilhafte Ausführungsformen sind in den abhängigen Ansprüchen gegeben.

Ein erfindungsgemässer Körperverband umfasst mindestens eine erste flüssigkeitsdurchlässige und/oder flüssigkeitsabsorbierende Innenschicht, mindestens eine zweite flüssigkeitsabsorbierende Zwischenschicht und mindestens eine dritte flüssigkeitsundurchlässige Aussenschicht. Der Körperverband ist einteilig ausgestaltet und dazu geeignet, einen Körperbereich eines Patienten zu umschliessen. Der Körperverband weist mindestens eine Öffnung auf, durch welche bei der vorgesehenen Benutzung des Körperverbandes ein Körperteil des Patienten ragen kann. An der mindestens einen Öffnung des Körperverbandes sind Abdichtungselemente vorgesehen, die dazu geeignet sind, den Körperverband gegenüber dem aus der Öffnung ragenden Körperteil des Patienten dichtend abzuschliessen.

Die Öffnungen des Körperverbandes weisen Abdichtungselemente auf, um die notwendige Dichtigkeit sicherzustellen, um einen Flüssigkeitsaustritt aus dem Körperverband zu verhindern. Dies hat den Vorteil, dass im restlichen Bereich eines erfindungsgemässen Körperverbandes die innere Oberfläche des Körperverbandes nicht bündig auf der Körperoberfläche aufliegen muss. Dies ermöglicht einen hohen Tragkomfort des Körperverbandes, da er locker geschnitten werden kann. Zudem kann er wie ein normales Kleidungsstück problemlos angelegt und bei Bedarf auch schnell gewechselt werden.

Es wird vorteilhaft vermieden, dass in neuralgischen Hautbereichen Druck- oder Klebestellen auftreten, insbesondere durch Verbindungs- bzw. Verschlusselemente, die eine unangenehme Druckwirkung ausüben können. Der Körperverband weist daher in einer vorteilhaften Ausführungsform eine Lockerheit auf, welche sich positiv auf die Atmungsaktivität des Körperverbandes auswirkt und welche dem Patienten ein hohes Mass an Tragkomfort und Beweglichkeit gewährt.

Die erste flüssigkeitsdurchlässige und/oder flüssigkeitsabsorbierende Innenschicht eines erfindungsgemässen Körperverbandes weist vorteilhaft eine dem Patienten zugewandte Oberfläche auf.

Diese der Patienten zugewandte Oberfläche dient dazu, die aus den Einschnittstellen auslaufende Flüssigkeit von der Patientenoberfläche in die weiteren Schichten des Körperverbandes zu leiten. Die aufgenommene Flüssigkeit wird von der ersten Schicht verteilt, sodass eine möglichst gleichmässige Verbreitung oder Absorption der Flüssigkeit innerhalb des Körperverbandes erreicht wird. Des Weiteren wird so eine lokale Durchnässung der Schichten verhindert.

In einer vorteilhaften Ausführung eines erfindungsgemässen Körperverbands ist die erste Innenschicht flüssigkeitsdurchlässig und aus einem Spinnvlies hergestellt.

Unter Spinnvlies werden hierbei Vliesstoffe verstanden, welche in einem kontinuierlichen Verfahren aus gesponnenen Fasern, bestehend aus synthetischen Polymeren, hergestellt werden. Die synthetischen Polymere werden als Granulat unterschiedlicher Geometrie extrudiert und gedehnt, um Filamente zu bilden. Diese Filamente werden dann durch eine aus Düsen austretenden Verstreckluft gekühlt und zu einem Vlies verlegt. Die Verbindung des Gewebes geschieht mittels thermischer, chemischer oder mechanischer Behandlung.

Die erfindungsgemässe zweite flüssigkeitsabsorbierende Zwischenschicht eines erfindungsgemässen Körperverbands besteht vorteilhaft aus einer schichtenweise verbundenen Kombinationsschicht aus schmelzgeblasenem flüssigkeitsabsorbierendem Vlies, Spinnvlies sowie einer abdeckenden Folie oder Membran.

Vorteilhaft ist die schmelzgeblasene Vliesschicht der Kombinationsschicht der zweiten flüssigkeitsabsorbierenden Zwischenschicht einer vom Patienten abgewandten Oberfläche der ersten flüssigkeitsdurchlässigen Innenschicht zugewandt.

Die von dem Patienten abgewandte Folie oder Membran verhindert, dass der Grossteil der absorbierten Flüssigkeit bis zur Aussenschicht des Körperverbandes gelangt und stellt dadurch ergänzend sicher, dass die Aussenschicht trocken bleibt.

Die zweite flüssigkeitsabsorbierende Zwischenschicht absorbiert die aus den Einschnittstellen auslaufende und durch die erste flüssigkeitsdurchlässige und/oder flüssigkeitsabsorbierende Innenschicht durchdringende Flüssigkeit und hält sie zurück. Ferner verteilt vorteilhaft die zweite flüssigkeitsabsorbierende Zwischenschicht die Flüssigkeit innerhalb des Körperverbandes.

Das Herstellungsverfahren eines schmelzgeblasenen Vliesstoffes unterscheidet sich von dem eines Spinnvliesstoffes vor allem dadurch, dass die Verstreckluft mit Kühlwirkung durch warme Luft ersetzt wird. Die Filamente werden also von der aus den Düsen in Austrittsrichtung der Filamente strömenden Luft verstreckt. So verbleiben die synthetischen Polymere länger in einem schmelzflüssigen Zustand als bei dem Spinnvlies-Herstellungsverfahren. Es können geringere Faserdurchmesser sowie weitere anwendungsspezifische Eigenschaften erreicht werden.

Die dritte flüssigkeitsundurchlässige Schicht eines erfindungsgemäss Körperverbands besteht vorteilhaft aus einer Folie, einer Laminatfolie oder Membran.

Bei einem erfindungsgemässen Körperverband bildet vorteilhaft die dritte flüssigkeitsundurchlässige Aussenschicht mit einer vom Patienten abgewandten Oberfläche die Aussenseite des Verbandes.

Insbesondere weist die Folie, laminierte Folie oder Membran eine vom Patienten abgewandte Oberfläche auf, welche die Aussenseite des Körperverbandes bildet. Die flüssigkeitsundurchlässige Aussenschicht dichtet den Körperverband nach aussen ab und verhindert einen Flüssigkeitsaustritt gegen aussen.

In einer möglichen Ausführungsform der vorliegenden Erfindung kann die dritte flüssigkeitsundurchlässige Schicht als laminierte Schmelzklebefolie ausgebildet werden.

Die erfindungsgemässe dritte flüssigkeitsundurchlässige Schicht kann verschiedene, vom Patienten wählbare, Farben annehmen.

Bei anderen vorteilhaften Ausführungsform eines erfindungsgemässen Körperverbands ist auf der flüssigkeitsundurchlässigen Aussenschicht aufliegend eine vierte Schicht aus textilem Gewebe angeordnet.

Diese zusätzlichen, stoffähnlichen vierten Schicht erhöht den Tragkomfort und kann zudem mit besonderen Designs ausgestattet sein. Die laminierte Schmelzklebefolie der dritten flüssigkeitsundurchlässigen Schicht besteht hierbei vorteilhaft aus einem Polymer-Material oder einem Material mit ähnlichen Eigenschaften.

Beim funktionalen Gebrauch des Körperverbandes, ist die als erste flüssigkeitsdurchlässige und/oder flüssigkeitsabsorbierende Innenschicht bezeichnete Schicht der Hautoberfläche des Patienten zugewandt, darauf folgt die zweite flüssigkeitsabsorbierende Zwischenschicht und anschliessend die dritte flüssigkeitsundurchlässige Aussenschicht. Die Innenschicht liegt vorteilhafterweise nicht unmittelbar auf der Haut auf, sondern die Form des Körperverbandes erlaubt einen gewissen, für den Patienten angenehmen, Abstand zwischen Haut und Innenschicht.

Der beschriebene Schichtenaufbau ermöglicht es, dass die Flüssigkeit bei solchen vorteilhaften Ausführungsformen nicht nur in distaler Richtung von der Hautoberfläche weggeführt bzw. absorbiert wird, sondern auch in Flächenrichtung des Vlies verteilt wird, um lokale Nassbereiche mit erhöhter Flüssigkeitsanreicherung möglichst zu vermeiden, da die dritte Schicht wie erwähnt eine dichtende Wirkung hat.

Erfindungsgemäss sind vorteilhaft die erste flüssigkeitsdurchlässige und/oder flüssigkeitsabsorbierende Innenschicht, die zweite flüssigkeitsabsorbierende Zwischenschicht und die flüssigkeitsundurchlässige Aussenschicht miteinander verbunden.

Der schichtenweise Verbund verhindert ein gegenseitiges Ablösen der jeweiligen Schichten. Dieser schichtenweise Verbund kann in einer vorteilhaften Ausführung durch diskontinuierlich verteilte Warmverklebungen realisiert werden. Eine weitere vorteilhafte Ausführung sieht einen Flächenverbund der jeweiligen Schichten vor, beispielsweise durch Verkleben.

Vorteilhaft sind die Materialien des erfindungsgemässen Körperverbandes so ausgestaltet, dass sie sterilisiert werden können. Beispielsweise ist ein erfindungsgemässer Körperverband vorteilhaft durch Strahlensterilisation und/oder trockenantiseptische Sterilisationsverfahren sterilisierbar.

Alternativ oder zusätzlich können einzelne oder mehrere Schichten eines erfindungsgemässen Körperverbandes antibakteriell ausgerüstet sein, beispielsweise mit elementarem Silber oder Silbersalzen. Eine antibakterielle Ausrüstung erlaubt es unter anderem, Geruchsentwicklungen zu verhindern

Ein erfindungsgemässer Körperverband ist vorteilhaft als einteiliges, den Körper umschliessendes Kleidungsstück ausgestaltet.

In Abhängigkeit der jeweiligen Körperbereiche des Patienten können die Kleidungsstücke angepasst werden, sodass Einschnittstellen komplett von dem Körperverband umschlossen werden.

Ein erfindungsgemässer Körperverband in Form eines einteiligen Kleidungsstücks weist vorteilhaft Verschlüsse auf.

Diese Verschlüsse können in Form von verschiedenen nicht dauerhaften Verschlusslösungen realisiert werden, zum Beispiel durch Reissverschluss oder Klettbandverschluss. Die Verschlüsse ermöglichen ein einfaches Anziehen und Ausziehen des Körperverbandes. Auch ein lösbar klebender Verschluss kann in alternativen Lösungen vorgesehen sein. Der Verschluss kann nach Bedarf an verschiedenen Stellen des Körperverbandes positioniert werden, in einer möglichen Ausführungsform bei Eingriffen in dem Bauch-, Arm- und/ oder Nackenbereich verläuft der Verschluss entlang der Längsachse zwischen dem Halsausschnitt und dem Taillenabschluss. Optional können mehrere breitere oder schmälere Verschlusslösungen verwendet werden für eine engere, bzw. breitere Passform sowie einen verbesserten Halt.

Die Abdichtungselemente an den Öffnungen des erfindungsgemässen Körperverbandes sind vorteilhaft als elastische oder unelastische Verengungen ausgebildet.

Diese Verengungen diesen dazu, die notwendige Dichtigkeit zu gewährleisten, um einen Flüssigkeitsauslauf aus dem Körperverband zu verhindern. Hierbei ist der Umfang der distalen Öffnungen der Abdichtungselemente ist in einem Ruhezustand kleiner als der Umfang der Öffnungen des postoperativen Körperverbandes, sodass die Öffnungen des postoperativen Körperverbandes und die Abdichtungselemente zusammen eine konische Verengung bilden. Die Abdichtungselemente können in Form von verschiedenen Abdichtungslösungen realisiert werden, inklusive, aber nicht limitiert auf, elastische Verengungen aus Baumwolle oder Kunststoff, unelastische Zugbänder, Schnallen, Schnüren, Haken, Klebe- und Klettbandverschlüssen. Besonders vorteilhaft wegen des Tragkomforts sind jedoch Baumwollabdichtungen mit oder ohne zusätzliche dichtende Aussenschicht.

Einige der bereits erwähnten möglichen Ausführungen der Abdichtungselemente sind verstellbar, sodass die Dichtigkeit in diesen Fällen von den Patienten kontrollierbar ist. Bei besonderen Ausführungsformen können am oder beim Übergang zwischen dem Vlies und den Abdichtungen vorteilhaft zusätzliche flüssigkeitsabsorbierende Verbandelemente eingenäht oder eingebracht sein, so dass einerseits der Tragkomfort optimiert wird und andererseits eine zusätzliche Flüssigkeitsaufnahme sichergestellt ist.

Gemäss einer weiteren vorteilhaften Ausführungsform sind die Abdichtungselemente an den Öffnungen des postoperativen Körperverbandes als elastische Verengungen aus abriebfester fusselarmer Baumwolle ausgebildet. Diese vorteilhafte Ausführungsform ist durch die abriebfeste fusselarme Baumwolle eine hygienische Lösung, da das Risiko einer Kontaminierung durch Reibung reduziert wird. Die Elastizität dieser Verengungen und die verwendete Baumwolle ermöglichen darüber hinaus den Patienten ein hohes Mass an Tragkomfort.

In einer anderen vorteilhaften Ausführungsform eines erfindungsgemässen Körperverbandes weist eine Öffnung des Körperverbandes für den Hals eines Patienten kein Abdichtungselement auf.

Stattdessen kann der Halsauschnitt abgenäht werden. Eine solche Lösung erhöht den Tragkomfort. Da im Stehen oder Sitzen Flüssigkeit von der Halsöffnung weg nach unten fliesst, hat dies keine Auswirkung auf die Dichtwirkung.

Der Körperverband kann Mittel aufweisen, mit welchen der Körperverband mit einem unter dem Körperverband zu tragenden Kompressionsverband reversibel verbindbar ist.

So wird eine verbesserte Positionierung und Halt des Körperverbandes an den behandelten Körperbereichen erzielt. Zum Beispiel ist in einer möglichen Ausführungsform der postoperative Körperverband nach einer Liposuktion im Unterkörperbereich des Patienten an dem Taillenabschluss mit dem Kompressionsverband reversibel verbindbar. Diese reversible Verbindung kann in möglichen Ausführungsformen durch einen Knopf- , Klammer-, Haken-, Schnur-, Klettverbund oder einen ähnlichen nicht dauerhaften Verbund realisiert werden.

Gemäss einer erfindungsgemässen Ausführungsform wird ein Körperverband nach einer Liposuktion im Hüft-, Taillen-, Schenkel-, Gesäss- und/oder Bauchbereich in Form einer Hose mit elastischen Verengungen an den Öffnungen vorgesehen. Die Hose ist einteilig ausgebildet, sodass sie die behandelten Körperbereiche umschliesst, wobei die elastischen Verengungen an der Öffnungen einen Auslauf von Flüssigkeiten aus dem postoperativen Körperverband verhindern. Des Weiteren weist die Hose eine Lockerheit auf (auch «loose fit» genannt), die den Patienten ein hohes Mass an Tragkomfort und Beweglichkeit gewährt.

Optional kann die Hose über einen Verschluss auf der Vorder- und/ oder Rückseite verfügen, um ein einfaches Anziehen und Ausziehen der Hose zu ermöglichen. Im Unterschied zum erwähnten Stand der Technik ist die Hose dabei grundsätzlich einteilig geschnitten und die Verschlüsse, soweit solche optional vorhanden sind, werden optimal positioniert, so dass keine Druckstellen bewirkt werden, wobei diese vorzugsweise von der Mitte versetzt, aber nicht direkt in den Hüftbereichen angeordnet sind.

Gemäss einer weiteren erfindungsgemässen Ausführungsform wird ein Körperverband nach einer Liposuktion im Bauch-, Arm- und/ oder Nackenbereich in Form eines Sweatshirts (in Form eines kurz- oder langarmigen Hemdes oder Pullovers) mit elastischen Verengungen an den Öffnungen, bzw. Ärmelenden und Taillenabschluss, vorgesehen. Der Halsausschnitt des Sweatshirts ist hierbei vorteilhaft nicht mit elastischer Verengung ausgestattet. Eine elastische Verengung im Halsbereich ist dannvorteilhaft, wenn ein operativer Eingriff im Nackenbereich erfolgte. Die Verengungen werden im Herstellungsverfahren vernäht oder dichtend mit dem Sweatshirt verbunden.

Das Sweatshirt ist einteilig ausgebildet, sodass es die behandelten Körperteile umschliesst, wobei die elastischen Verengungen an den Öffnungen einen Auslauf von Flüssigkeiten aus dem postoperativen Körperverband verhindern. Bei besonderen Ausführungsformen können die Verengungen zusätzlich mit einer dichtenden Schicht überzogen oder aus einem dichtenden Material hergestellt sein. Optional können auch die Verbindungsnähte oder Befestigungen dichtend überzogen sein.

Des Weiteren weist das Sweatshirt, ähnlich wie bei der Hose beschrieben, eine Lockerheit auf, die den Patienten ein hohes Mass an Tragkomfort und Beweglichkeit gewährt. Optional kann das Sweatshirt über einen Verschluss auf der Vorder- und/ oder Rückseite verfügen, um ein einfaches Anziehen und Ausziehen des Sweatshirts zu ermöglichen. Auch dieser Verschluss wird vorteilhaft so ausgebildet, dass er keine Druck- oder Pressstellen bewirkt.

### Figurenbeschrieb

Die im Folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.
- Figur 1: zeigt eine schematische Darstellung eines erfindungsgemässen Körperverbandes in einer vorteilhaften Ausführungsform für die postoperative Behandlung nach Liposuktionen im Hüft-, Taillen-, Gesäss- und/oder Bauchbereich.
- Figur 2: zeigt eine schematische Darstellung einer Verwendung des Körperverbandes aus Figur 1 an einem Körper eines Patienten mit Kompressionsverband.
- Figur 3: zeigt eine schematische Darstellung eines erfindungsgemässen Körperverbandes in einer vorteilhaften Ausführungsform für die postoperative Behandlung nach Liposuktionen am Oberkörper des Patienten.
- Figur 4: zeigt schematisch einen Schichtenaufbaus des erfindungsgemässen Körperverbandes aus Figur 3, in einem voneinander gelösten Zustand.
- Figur 5: zeigt eine Darstellung der Innenseite eines Verbundvliesstoffes gemäss einer vorteilhaften Ausführungsform der vorliegenden Erfindung mit Linienverschweissung.

### Ausführung der Erfindung

Falls nicht ausdrücklich gegenteilig angegeben oder sich lösungsmässig ausschliessend, können die Lösungen gemäss der Erfindung nach Wunsch kombiniert und durch die weiteren folgenden Ausführungsformen, die in sich selbst vorteilhaft sind, jeweils weiter verbessert werden.

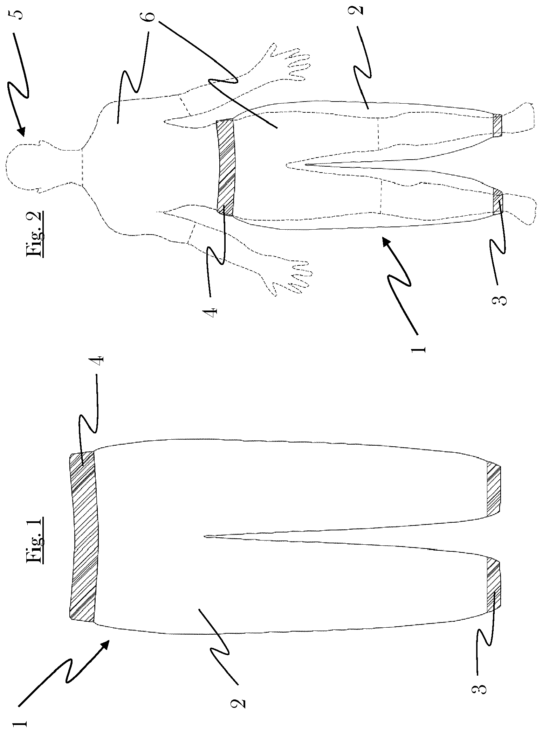

Figur 1 zeigt eine erfindungsgemässe Ausführungsform des vorliegenden Körperverbandes 1, insbesondere für die postoperative Behandlung nach Liposuktionen im Bein-, Gesäss-, Hüft-, Taillen- und/oder Bauchbereich. Der postoperative Körperverband 1 bildet in dieser beispielhaften Ausführungsform eine Hose 2, welche an den Öffnungen bzw. Hosenbeinenden und am Taillenabschluss Abdichtungselemente 3, 4, beispielsweise elastische Verengungen aus abriebfester fusselarmer Baumwolle, aufweist. Die Hose 2 ist so ausgestaltet, dass der gesamte Unterkörper und die Beine des Patienten umschlossen werden, wobei nur die elastischen Verengungen an den Hosenbeinenden und dem Taillenabschluss einen Druck auf den Körper ausüben. Der postoperative Körperverband 1 in Form einer Hose 2 gemäss Figur 1 weist (abgesehen von der Bundöffnung und den Beinöffnungen) keine separaten Öffnungen mit Verschluss auf, da die elastischen Verengungen ein einfaches Anziehen und Ausziehen ermöglichen. Die Hose 2 ist so geschnitten, dass sie einen lockeren, aber nicht zu weiten Sitz über der Haut des Patienten bewirkt. Dadurch wird ein Traggefühl ähnlich einer langen Trainingshose bewirkt, was anhand der nachfolgenden Abbildung näher beschrieben ist.

Figur 2 ist eine Darstellung eines Patienten 5 mit Kompressionsverband 6, wobei der Patient 5 den Körperverband 1 gemäss Figur 1 oberhalb des Kompressionsverbandes 6 trägt. Die Abdichtungselemente 3, 4, beispielsweise elastischen Verengungen sind formpassend ausgestaltet, sodass die austretende Flüssigkeit von den inneren Schichten 100, 200 des postoperativen Körperverbandes 1 aufgenommen wird und nicht aus den Abdichtungselementen 3, 4, bzw. elastischen Verengungen ausläuft. Nur am Bund und den unteren Beinenden liegt die Hose 2 direkt auf der Haut des Patienten auf. In den übrigen Bereichen ist der Schnitt lose sitzend.

Die in Figur 1 und Figur 2 abgebildeten Ausführungsformen der vorliegenden Erfindung können in Kombination mit Kompressionsverbänden 6 getragen werden, um eine optimale postoperative Pflege zu erzielen. Der Patient zieht hierbei unmittelbar nach einer Liposuktion den Kompressionsverband 6 über die behandelten Körperteile gemäss den Instruktionen des zuständigen Arztes. Daraufhin wird der Körperverband 1, 10 über dem Kompressionsverband angezogen, wobei die Abdichtungselemente 3, 4, 30, 40 sowie die Lockerheit des Körperverbandes 1, 10 ein einfaches Anziehen und Ausziehen begünstigen.

Es ist im Gebiet der Liposuktion vorteilhaft, den Kompressionsverband 6 nach einem Eingriff kontinuierlich zu tragen, um den Heilungsprozess zu fördern und das Entzündungsrisiko zu minimieren.

Gemäss einer weiteren möglichen Ausführungsform ist der Körperverband 1, 10 reversibel verbindbar mit dem Kompressionsverband 6. Dies erlaubt dem Patienten eine stabile Positionierung des Körperverbandes 1, 10. Der Kompressionsverband 6 kann dennoch kontinuierlich getragen werden, wenn der Körperverband 1, 10 ausgewechselt werden muss. Der Körperverband 1, 10 kann auch nach dem Ende der Kompressionstherapie getragen werden, falls Patienten weiterhin mit einem postoperativen Flüssigkeitsauslauf bekümmert sind. Der Kompressionsverband 6 ist regelmässig waschbar, wohingegen der erfindungsgemässe Körperverband 1, 10 nach einer gewissen Tragzeit z.B. einem Tag aus hygienischen Gründen sowie wegen des Tragkomforts entsorgt wird.

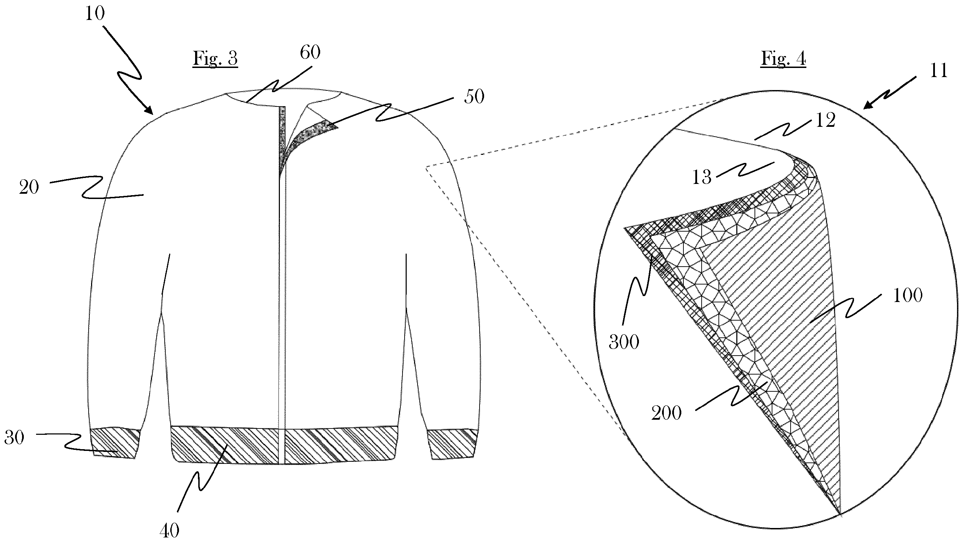

Figur 3 zeigt eine erfindungsgemässe Ausführungsform des vorliegenden postoperativen Körperverbandes 10, insbesondere für die postoperative Behandlung nach Liposuktionen im Bauch-, Taillen-, Arm- und/ oder Nackenbereich. Der Körperverband 10 bildet in dieser vorteilhaften Ausführungsform ein Sweatshirt 20, welches an den Öffnungen, bzw. Ärmelenden und am Hüftenabschluss, Abdichtungselemente 30, 40, beispielsweise elastische Verengungen aus abriebfester fusselarmer Baumwolle aufweist. Das Sweatshirt 20 ist gemäss einer vorteilhaften Ausführungsform der vorliegenden Erfindung mit einem frontalen Verschluss 50 ausgebildet, beispielsweise als Klettverschluss oder Reissverschluss.

Der Halsauschnitt (60) des Sweatshirts 20 ist in Figur 3 abgenäht, kann jedoch gemäss einer weiteren möglichen Ausführungsform ebenfalls Abdichtungselemente 3, 4 aufweisen.

Der dargestellte Körperverband 10 gemäss Figur 3 kann auch ohne frontalen Verschluss 50 ausgeführt sein. In diesem Fall wird er wie ein Pullover angezogen.

Die in Figur 1 und Figur 3 abgebildete Ausführungsformen können auch ohne vom Anwendungsbereich der Erfindung abzuweichen in ärmelloser, halbärmeliger und langärmeliger oder anderen zweckmässigen Formen ausgestaltet sein.

Figur 4 zeigt den Schichtenaufbau des Verbundvliesstoffes 11 des erfindungsgemässen Körperverbandes 1, 10, mit einer dem Patienten zugewandten Innenseite 12 und einer dem Patienten abgewandten Aussenseite 13, in einem unverbundenen Zustand. Der Schichtenaufbau in Figur 4 von der Innenseite 12 zu der Aussenseite 13 betrachtet umfasst eine erste flüssigkeitsdurchlässige und/oder flüssigkeitsabsorbierende Innenschicht 100, eine zweite flüssigkeitsabsorbierende Zwischenschicht 200 und eine flüssigkeitsundurchlässige Aussenschicht 300. Eine besonders gute postoperative Wirkung wird erzielt, wenn die erste Schicht flüssigkeitsdurchlässig ist, insbesondere aus einer semipermeablen Schicht oder eine Porenschicht besteht, über der die zweite absorptionsfähige Schicht, insbesondere ein saugendes Vlies, liegt.

Die in Figur 4 abgebildete Aussenseite 13 der flüssigkeitsundurchlässigen Aussenschicht 300 kann gemäss möglichen Ausführungsformen der vorliegenden Erfindung verschiedene, von den Patienten wählbare, Farben besitzen, um den Patienten eine ästhetisch attraktive Wahl anbieten zu können. Auch eine Bedruckung mit Mustern ist möglich. Die Lösungen aus dem Stand der Technik hingegen haben eine medizinische Ästhetik.

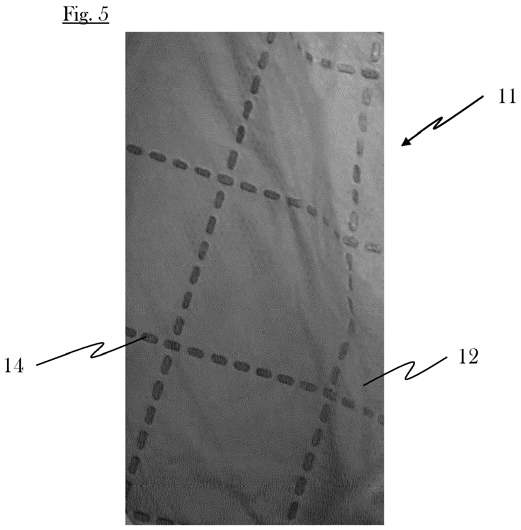

In Figur 5 ist die Innenseite 12 des Verbundvliesstoffes 11 gemäss einer vorteilhaften Ausführungsform der vorliegenden Erfindung abgebildet. Der schichtenweise Verbund der ersten flüssigkeitsdurchlässigen und/oder flüssigkeitsabsorbierenden Innenschicht 100, der zweiten flüssigkeitsabsorbierenden Zwischenschicht 200 und der flüssigkeitsundurchlässigen Aussenschicht 300 werden in Figur 5 durch sich im Abstand kreuzende diskontinuierliche Linienverschweissung 14 verbunden, die vorteilhaft mittels Thermoschweissen oder alternativ durch Ultraschallschweissen angebracht werden. Auf diese Weise wird eine optimale Flüssigkeitsabsorption bewirkt und eine einfache Herstellbarkeit bei angemessenen Kosten erzielt.

Besonders vorteilhaft werden durch die Schweissung saugfähige Bauschbereiche in Form von Parallelogrammen mit Seitenlängen von annähernd 10x10 cm, vorteilhaft von annähernd 5x5 cm und besonders vorteilhaft von annähernd 3x3 cm erzeugt. Die Schweissungen weisen eine möglichst schmale Linienbreite auf, besonders vorteilhaft von weniger als 1.5 mm.

Bei alternativen Ausführungsformen haben sich auch matrizenförmige Schweissungen als geeignet erwiesen, bei denen eine Schweissung durch geschlossene Geometrien vorgesehen werden, z.B. kreis- oder ellipsenförmige Schweissungen, die in Abständen voneinander angebracht sind.

Solche matrizenförmige Schweissungen werden gemäss einer weiteren möglichen Ausführungsform durch Rotationsschweissvorrichtungen aufgebracht, wobei das Schichtmaterial kontinuierlich durch die Vorrichtung geführt wird. Auf diese Weise lassen sich nicht nur die Schweissnähte verringern, wobei die flüssigkeitsabsorbierenden Bauschbereiche optimiert angeordnet werden können, sondern es können auch gezielte designtechnische Effekte bewirkt werden, z.B. Ringe, Logos und Weiteres.

Die in der vorliegenden Erfindung offenbarte Mehrschichtenlösung ist nicht auf die im Figurenbeschrieb erläuterten drei Schichten beschränkt, sondern kann, ohne vom Anwendungsbereich der vorliegenden Erfindungen abzuweichen, weitere Schichten enthalten. Je nach Bedarf kann hier eine weitere absorptionsfähige, atmungsaktive, flüssigkeitsundurchlässige oder sonstige funktionale Schicht miteinbezogen werden. Weiterhin kann über der dichtenden dritten Schicht auch eine weitere Schicht, insbesondere eine Stoffschicht, vorgesehen sein, die den Tragkomfort erhöht und es dem Patienten erlaubt, auf eine zusätzliche Überkleidung zu verzichten.

Die vorliegende Erfindung ist in ihrem Umfang nicht auf die hier beschriebenen spezifischen Ausführungsformen beschränkt. Vielmehr ergeben sich für den Fachmann aus der Beschreibung und den dazugehörigen Figuren zusätzlich zu den hier offenbarten Beispielen verschiedene weitere Modifikationen der vorliegenden Erfindung, die ebenfalls in den Schutzbereich der Ansprüche fallen.

### Bezugszeichenliste

- 1: Postoperativer Körperverband
- 2: Postoperativer Körperverband als Hose ausgebildet
- 3: Elastische Verengung am Beinabschluss der Hose
- 4: Elastische Verengung am Taillenabschluss der Hose
- 5: Patient
- 6: Kompressionsverband
- 10: Postoperativer Körperverband
- 20: Postoperativer Körperverband als Sweatshirt ausgebildet
- 30: Elastische Verengung am Ärmelabschluss des Sweatshirts
- 40: Elastische Verengung am Taillenabschluss des Sweatshirts
- 50: Verschluss
- 60: Halsauschnitt
- 11: Verbundvliesstoff
- 12: Innenseite des Verbundvliesstoffes
- 13: Aussenseite des Verbundvliesstoffes
- 14: Linienverschweissung
- 100: Erste absorptionsfähige Innenschicht
- 200: Zweite absorptionsfähige Zwischenschicht
- 300: Dritte flüssigkeitsundurchlässige Aussenschicht

## Patentansprüche

1. Körperverband (1, 10), umfassend
mindestens eine erste flüssigkeitsdurchlässige und/oder flüssigkeitsabsorbierende Innenschicht (100),
mindestens eine zweite flüssigkeitsabsorbierende Zwischenschicht (200) und
mindestens eine dritte flüssigkeitsundurchlässige Aussenschicht (300);
**dadurch gekennzeichnet, dass** der Körperverband einteilig ausgestaltet ist und dazu geeignet ist, einen Körperbereich eines Patienten zu umschliessen;
wobei der Körperverband mindestens eine Öffnung aufweist, durch welche bei der vorgesehenen Benutzung des Körperverbandes ein Körperteil des Patienten ragen kann; und
wobei an der mindestens einen Öffnung des Körperverbandes Abdichtungselemente (3, 4, 30, 40) vorgesehen sind, die dazu geeignet sind, den Körperverband gegenüber dem aus der Öffnung ragenden Körperteil des Patienten dichtend abzuschliessen.

2. Körperverband gemäss Anspruch 1, wobei die erste flüssigkeitsdurchlässige Innenschicht (100) eine dem Patienten zugewandte Oberfläche aufweist.

3. Körperverband gemäss Anspruch 1 oder 2, wobei die erste Innenschicht (100) flüssigkeitsdurchlässig ist und aus einem Spinnvlies hergestellt ist.

4. Körperverband gemäss einem der Ansprüche 1 bis 3, wobei die zweite flüssigkeitsabsorbierende Zwischenschicht (200) als eine Kombinationsschicht von schmelzgeblasenem Vlies, Spinnvlies und einer abdeckenden Folie oder Membran hergestellt ist.

5. Körperverband gemäss Anspruch 4, wobei die schmelzgeblasene Vliesschicht der Kombinationsschicht der zweiten flüssigkeitsabsorbierenden Zwischenschicht einer vom Patienten abgewandten Oberfläche der ersten flüssigkeitsdurchlässigen Innenschicht zugewandt ist.

6. Körperverband gemäss einem der vorgenannten Ansprüche, wobei die flüssigkeitsundurchlässige Aussenschicht (300) aus einer Folie, Laminatfolie oder Membran besteht.

7. Körperverband gemäss einem der vorgenannten Ansprüche, wobei die flüssigkeitsundurchlässige Aussenschicht (300) mit einer vom Patienten abgewandten Oberfläche die Aussenseite (13) des Verbandes bildet.

8. Körperverband gemäss einem der vorgenannten Ansprüche, wobei auf der flüssigkeitsundurchlässigen Aussenschicht aufliegenden eine vierten Schicht aus textilem Gewebe angeordnet ist.

9. Körperverband gemäss einem der vorgenannten Ansprüche, wobei die erste flüssigkeitsdurchlässige Innenschicht (100), die zweite flüssigkeitsabsorbierende Zwischenschicht (200) und die flüssigkeitsundurchlässige Aussenschicht (300) miteinander verbunden sind.

10. Körperverband gemäss einem der vorgenannten Ansprüche, wobei der Körperverband als einteiliges, den Körper umschliessendes Kleidungsstück ausgestaltet ist.

11. Körperverband gemäss Anspruch 10, wobei der als einteiliges Kleidungsstück ausgestaltete Körperverband Verschlüsse (50) aufweist.

12. Körperverband gemäss einem der vorgenannten Ansprüche, wobei Abdichtungselemente (3, 4, 30, 40) an den Öffnungen des Körperverbandes als elastische oder unelastische Verengungen ausgebildet sind.

13. Körperverband gemäss einem der vorgenannten Ansprüche, wobei eine Öffnung des Körperverbandes für den Hals eines Patienten kein Abdichtungselement aufweist.

14. Körperverband gemäss einem der vorgenannten Ansprüche, wobei der Körperverband Mittel aufweist, mit welchen der Körperverband mit einem unter dem Körperverband zu tragenden Kompressionsverband reversibel verbindbar ist.
